(19)

Europäisches
Patentamt
European
Patent Office
Office européen
des brevets

(11) **EP 2 117 512 B1**

(12) **EUROPEAN PATENT SPECIFICATION**

(45) Date of publication and mention
of the grant of the patent:
**28.02.2018 Bulletin 2018/09**

(21) Application number: **08717845.5**

(22) Date of filing: **14.03.2008**

(51) Int Cl.:
***A61K 9/14*** *(2006.01)*

(86) International application number:
**PCT/EP2008/053104**

(87) International publication number:
**WO 2008/110626 (18.09.2008 Gazette 2008/38)**

(54) **STABILIZED MICRONISED PARTICLES**

STABILISIERTE MIKRONISIERTE PARTIKEL

DES PARTICULES MICRONISÉES STABLES

(84) Designated Contracting States:
**AT BE BG CH CY CZ DE DK EE ES FI FR GB GR
HR HU IE IS IT LI LT LU LV MC MT NL NO PL PT
RO SE SI SK TR**

(30) Priority: **15.03.2007 EP 07104248**

(43) Date of publication of application:
**18.11.2009 Bulletin 2009/47**

(73) Proprietor: DSM IP Assets B.V.
**6411 TE Heerlen (NL)**

(72) Inventors:
• **HEE, VAN, Pim**
**NL-2625 LJ Delft (NL)**
• **MEESTERS, Gabriel Marinus Henricus**
**NL-2614 TJ Delft (NL)**
• **WILDEBOER, Willem Johannes**
**2543 TB Den Haag (NL)**
• **HENNART, Stéphen L. A.**
**NL-2286 HT Rijswijk (NL)**
• **VIS, Albert-Jon**
**NL-3039 WL Rotterdam (NL)**

(74) Representative: **DSM Intellectual Property**
**P.O. Box 4**
**6100 AA Echt (NL)**

(56) References cited:
**EP-A- 0 678 241      EP-A2- 0 807 431
WO-A-91/06292      WO-A-2007/014566
GB-A- 1 148 801      US-A- 4 436 523
US-A- 4 474 872      US-B1- 6 413 537**

• **T Yokoyama ET AL: "Nanoparticle Technology
for the Production of Functional Materials",
KONA., vol. 23, 1 January 2005 (2005-01-01),
pages 7-17, XP055093752, JP ISSN: 0288-4534,
DOI: 10.14356/kona.2005006**
• **DAN LI AND RICHARD B KANER*: "Shape and
Aggregation Control of Nanoparticles: Not
Shaken, Not Stirred", JOURNAL OF THE
AMERICAN CHEMICAL SOCIETY, AMERICAN
CHEMICAL SOCIETY, US , vol. 128, no. 3 25
January 2006 (2006-01-25), pages 968-975,
XP008177790, ISSN: 0002-7863, DOI:
10.1021/JA056609N Retrieved from the Internet:
URL:http://pubs.acs.org/doi/pdf/10.1021/ja
056609n [retrieved on 2005-12-31]**

## Description

### Field of the invention

The present invention relates to a method for particle size reduction, in particular to a method for particle size reduction of organic compounds.

### Background of the invention

Various methods for particle size reduction are known. These methods involve size reduction of dry powders and liquid-suspended particles. In most cases the latter category makes use of stabilizers to prevent aggregation of the suspended particles. The stabilizers consist of surfactants, thickeners e.g. hydrocolloids, and/or other agents that impact the interaction between the particles and the movement of the particles in the liquid/fluid phase. When the particles are sufficiently large, sedimentation of liquid-suspended particles may occur. In order to prevent this phenomenon, the viscosity of the liquid phase is increased by addition of stabilizers such as thickeners e.g. hydrocolloids and other soluble polymers.

Addition of thickeners can however not fully prevent sedimentation of large particles. The particles will thus eventually form sediment. This is a major disadvantage for application of the particle suspensions because 1) the sediment is often not so easily resuspended, and 2) the particles are not homogeneously distributed throughout the suspension.

Sedimentation will not occur at all when the particles are very small. It is thus advantageous to reduce the particle size. Nevertheless, small particles can easily form aggregates that will settle when the aggregates are large enough. Particle aggregation thus needs to be prevented in these systems. One approach is to formulate the suspension immediately after grinding. However, this is not possible in all applications. Another approach is to use additives such as stabilizers.

WO 98/35666 describes how naproxen obtained from the supplier in powder form is made into nanoparticulate form by grinding techniques in the presence of a surface modifier.

WO 91/06292 describes a process of preparing a hydrophobic/aerophilic solid which can be dispersed in water in the form of discrete microparticles, wherein the solid is milled in an aqueous medium in the presence of a hydrocolloid to obtain a suspension of microparticles.

US 2,876,160 describes how physical stability of a water-insoluble material is safe-guarded by embedding the material in chemical modified starch.

In US 4,006,025 a substantially water-insoluble spectral sensitizing dye is dispersed in water and then milled, or preferably homogenized, at an elevated temperature in the presence of a surfactant.

US 5,858,410 describes a method for preparing nanosuspensions by cavitation or shearing and impact forces in the presence of surfactants or thickeners.

WO 2007/014566 describes suspensions comprising crystalline particles of Coenzyme Q10 dispersed in an aqueous solution of a matrix material such as naturally occuring or modified polysaccharides, naturally occuring hydrocolloids, starch derived from a natural source or modified starch.

EP 0807431 describes a process for the manufacture of carotenoid compositions comprising surfactant.

GB 1,148,801 describes stabilised nystatin compositions comprising colloidal silica.

Yokoyama T. et al. (KONA No. 23 (2005), 7-17) describes that particle size can be reduced by pH adjustment but is silent about the effects thereof on stablity.

Aggregation -and as a consequence thereof sedimentation- of small particles is often prevented by addition of stabilizers. However, as indicated above the addition of these compounds frequently does not suffice to achieve prevention of aggregation and sedimentation of the particles. Moreover, the addition of stabilizers may be unwanted in certain applications, such as applications wherein stabilizer-free compositions are more suitable, stabilizers are not allowed because of legislation and/or stabilizers are not compatible with the active compound. Furthermore, the addition of a stabilizer may lead to higher costs and may thus be economical unattractive. In addition, th use of a stabilizer may be unattractive from an environmental point of view.

In view thereof, there exists a need for stable compositions of small particles that have good sedimentation properties and that are substantially free of stabilizers.

### Detailed description of the invention

The present invention is directed to a method for preparing a stable suspension of micronised particles of natamycin in the absence of a stabilizer, the method comprising the steps of: a) subjecting the natamycin to wet grinding at distinct pH values to obtain suspensions of micronised particles of natamycin each having a distinct pH value, or subjecting natamycin to wet grinding at one pH value and adjusting the pH of the suspension after grinding to obtain suspensions of micronised particles of natamycin each having a distinct pH value, b) measuring the zeta potential of the obtained suspensions and determining which of the obtained suspensions has a zeta potential of 10 mV or higher or -20 mV or lower, and c) preparing a suspension of micronised particles of natamycin by subjecting the natamycin to size reduction by wet grinding under pH conditions, wherein the suspension has a zeta potential of 10 mV or higher or -20 mV or lower according to step b). The method comprises subjecting natamycin to size reduction by wet grinding under pH conditions wherein the intrinsic surface charge of the size-reduced organic compound is sufficient to keep the suspension stable. In an embodiment the suspension made according to a method according to the invention is stable and substantially stabilizer-free. To put it differently, the suspension is stable under substantially stabilizer-free conditions. The stable suspension containing the size reduced particles can be made by wet grinding in the absence of a substantial amount of stabilizer (stability increasing additive). A stabilizer is not needed before, during and/or after the applied size reduction technique (*e.g.* wet grinding) to keep the suspension stable. It is clear for the person skilled in the art that "substantially" in this respect means that the suspension may contain a stabilizer, but that if the suspension comprises a stabilizer the amount thereof in the suspension is too low to have any significant stabilizing effect on the suspension. In an embodiment the suspension is free of any stabilizer.

An aspect of the invention relates to a method for preparing a stable suspension of micronised particles of natamycin in the absence of a stabilizer, the method comprising the steps of a) subjecting the solid organic compound to wet grinding at distinct pH values to obtain suspensions of micronised particles of the solid organic compound each having a distinct pH value; b) determining which of the obtained suspensions is stable; and c) preparing a stable suspension of micronised particles of the solid organic compound by subjecting the solid organic compound to size reduction by wet grinding under pH conditions, wherein the suspension is stable according to step b. In step a the solid organic compound may be suspended in distinct liquids or super critical fluids containing one or more buffering agents and having distinct pH values and then each suspension may be subjected to wet grinding to obtain suspensions of micronised particles of the solid organic compound. Alternatively, in step a the solid organic compound may be suspended in a liquid or supercritical fluid having one pH value, *e.g.* water or a solution having a buffering agent, and either before wet grinding or during wet grinding the pH of the suspension is adjusted, *e.g.* by addition of an acid, base or buffering agent, to distinct pH values. Either way, wet grinding at distinct pH values/conditions is accomplished. Of course, wet grinding of the solid organic compound at distinct pH values in step a can be performed simultaneously, but can also be performed consecutively. To find optimal conditions for the preparation of a stable suspension of micronised particles of a solid organic compound in the absence of a stabilizer, steps a and b can optionally be repeated at least once.

In another aspect the invention provides a suspension of micronised particles of natamycin, obtainable by the method according to anyone of the claims 1 to 7. The method comprising the steps of a) subjecting the solid organic compound to wet grinding at one pH value and adjusting the pH of the suspension after grinding to obtain suspensions of micronised particles of the solid organic compound each having a distinct pH value; b) determining which of the obtained suspensions is stable; and c) preparing a stable suspension of micronised particles of the solid organic compound by subjecting the solid organic compound to size reduction by wet grinding under pH conditions, wherein the suspension is stable according to step b. In step a the solid organic compound may be suspended in a liquid or supercritical fluid having one pH value and may then be subjected to wet grinding at this pH value to obtain a suspension of micronised particles. Next, samples of the suspension of micronised particles (which has one pH value) are taken and the pH of each sample is adjusted to a distinct pH value with a suitable compound, e.g. an acid, base or buffering agent, to obtain suspensions each having a distinct pH value. To find optimal conditions for the preparation of a stable suspension of micronised particles of a solid organic compound in the absence of a stabilizer, steps a and b can optionally be repeated at least once.

In another aspect the invention relates to a method for preparing a stable suspension of micronised particles of a solid organic compound in the absence of a stabilizer, the method comprising the steps of a) suspending the solid organic compound in a liquid or supercritical fluid; b) measuring the zeta potential of the suspension at distinct pH values; and c) preparing a stable suspension of micronised particles of the solid organic compound by subjecting the solid organic compound to size reduction by wet grinding under pH conditions, wherein the suspension is stable according to step b. The distinct pH values can be achieved by suspending the solid organic compound in liquids or supercritical fluids, each liquid or fluid having a distinct pH value. Alternatively, the solid organic compound can be suspended in a liquid or supercritical fluid having one pH value and the distinct pH conditions are created in the apparatus measuring the zeta potential of the obtained suspension (the zeta potential is measured at a pH profile).

Methods known in the art including determining the zeta potential of the suspensions comprising micronised particles of the solid organic compound; determining sedimentation of micronised particles in the suspensions in time; or determining the size of micronised particles in the suspensions in time can be used to determine which of the obtained suspensions are stable. Of course, a combination of these methods can also be used.

The pH conditions/values used in step a or b of a method according to the invention are preferably between pH 1 and 14. "Distinct" as used herein means several suspensions, each suspension having a different pH, for instance, a suspension having a pH of 2, a suspension having a pH of 3, etc. Preferably, at least 2, 3, 4, 5, 6, 7, 8, 9, 10, 11, 12, 13 or 14 different suspensions, each suspension having a distinct pH value between pH 1 and 14 are prepared. Preferably, suspensions having at least one pH value per pH unit are included.

In step b the stability of the suspensions obtained in step a is determined, *i.e.* it is determined which suspension is stable and which suspension is not stable. So, step b may include the step of determining the stability of the suspensions and on the basis thereof determining/assessing which suspensions are stable and which are not stable. Suitable methods to determine/measure the stability include the methods given above. Of course, other methods to determine/measure the stability of suspensions containing particles are also included in the present invention. A person skilled in the art is aware of other suitable methods.

When zeta potential measurements are used for determining the stability, suspensions are considered stable in the context of the present invention when the zeta potential as measured by dynamic light scattering as set forth herein is e.g. at least 10 mV or higher or e.g. at least -10 mV or lower. Preferably, the zeta potential is at least 15 mV or higher, at least 20 mV or higher, at least 25 mV or higher, at least 30 mV or higher, at least 35 mV or higher, at least 40 mV or higher, at least 45 mV or higher, at least 50 mV or higher, at least 55 mV or higher, and most preferably at least 60 mV or higher. Alternatively, the zeta potential may also preferably be at least -15 mV or lower, at least -20 mV or lower, at least -25 mV or lower, at least -30 mV or lower, at least -35 mV or lower, at least -40 mV or lower, at least -45 mV or lower, at least -50 mV or lower, at least -55 mV or lower and most preferably at least -60 mV or lower.

When sedimentation of micronised particles of the suspensions is used as a method to determine the stability of suspensions, suspensions are considered stable in the context of the present invention when e.g. the percentage below the sedimentation front measured as set forth herein is at least 70%, at least 75%, at least 80%, at least 85%, at least 90%, at least 95% after 1, 2, 3, 4, 5, 6, 7, and preferably 8 days (after grinding).

When the size of the particles in the suspensions is determined to get an indication of the stability of the suspensions, suspensions are considered stable in the context of the present invention when e.g. the mean particle size as given by the Stokes formula for hindered settling in a laminar flow does not increase more than a factor 10, 9, 8, 7, 6, 5, 4, 3, 2, more preferably the mean particle size increase should be less than a factor 1.5, 1.3, 1.2, 1.1, 1.05, 1.01, 1.005, 1.001, over a period of at least 1, 2, 3, 4, 5, 6, 7, 8, 9, 10, 15, 20, 25, or 30 days after size reduction, *e.g.* by grinding. In a preferred embodiment the mean particle size does not increase at all over the period indicated. The Stokes formula is as follows:

$$D_{stokes} = \sqrt{\frac{V_s \cdot 18 \cdot \mu_{fluid}}{g \cdot \left(\rho_{particle} - \rho_{fluid}\right)}}$$

$D_{stokes}$: Stokes diameter of particle
$V_s$: Initial settling velocity determined experimentally
$\mu_{fluid}$: viscosity of the liquid phase
g: gravity
$\rho_{fluid}$: density of the liquid phase
$\rho_{particle}$: density of the product particle

In the above formula the Stokes diameter of particle, as resulting from the hydrodynamic behaviour of the particle in a fluid, is qualified as equivalent to the diameter of a spherical particle having the same hydrodynamic behaviour (see e.g. Camp T.R. (1946), Sedimentation and the design of settling tanks, Trans. Am. Soc. Civ. Eng. 111:895-958; Dobbins W.E. (1944), Effect of turbulence on sedimentation. Trans. Am. Soc. Civ. Eng. 109:629-653).

Alternatively, suspensions are considered stable in the context of the present invention when the mean particle size ($d_{4,3}$) as determined by Laser Diffraction Analysis does not increase more than a factor 10, 9, 8, 7, 6, 5, 4, 3, 2, more preferably the mean particle size increase should be less than a factor 1.5, 1.3, 1.2, 1.1, 1.05, 1.01, 1.005, 1.001, over a period of at least 1, 2, 3, 4, 5, 6, 7, 8, 9, 10, 15, 20, 25, or 30 days after size reduction, *e.g.* by grinding. In a preferred embodiment the mean particle size does not increase at all over the period indicated.

On the basis of the above methods, pH values/conditions suitable for grinding are chosen. For instance, pH values/conditions wherein the suspensions are stable, *e.g.* pH values/conditions of suspensions that have a zeta potential that is at least -10 mV or lower or wherein the percentage below the sedimentation front measured as set forth herein is at least 70% after 8 days (after grinding) can be applied during the wet grinding in step c of a method of the invention to prepare a stable suspension of micronised particles of the solid organic compound. A stable suspension of micronised particles can already be produced in step b, if one of the suspensions tested is stable. Nevertheless, steps a and b are predominantly directed to small scale preparations and testing of suspension stabilities. A person skilled in the art will understand that testing the stability of suspensions will not be done on a large, *e.g.* industrial, scale. Step c is directed to the actual preparation of the stable suspensions, *e.g.* industrial scale, preparation.

In an embodiment the present invention provides for a method for preparing a stable suspension of micronised particles of a solid organic compound in the absence of a stabilizer according to the invention, the method comprising the step of *e.g.* subjecting the solid organic compound to size reduction by wet grinding under pH conditions wherein the zeta potential of the size-reduced organic compound is 10 mV or higher or -10 mV or lower. Under these zeta potential conditions the suspensions are found to be stable. *Ergo,* in an embodiment the size reduction by wet grinding is done in the absence of a stabilizer under pH conditions, wherein the zeta potential of the size-reduced organic compound is 10 mV or higher. Preferably, the zeta potential of the size-reduced organic compound is 15 mV or higher, 20 mV or higher, 25 mV or higher, 30 mV or higher, 35 mV or higher, 40 mV or higher, 45 mV or higher, 50 mV or higher, 55 mV or higher, and most preferably 60 mV or higher. In another embodiment, the size reduction by wet grinding is done in the absence of a stabilizer under pH conditions, wherein the zeta potential of the size-reduced organic compound is -10 mV or lower. Preferably, the zeta potential of the size-reduced organic compound is -15 mV or lower, -20 mV or lower, -25 mV or lower, -30 mV or lower, -35 mV or lower, -40 mV or lower, - 45 mV or lower, -50 mV or lower, -55 mV or lower and most preferably -60 mV or lower.

The main advantage of the method according to the invention is that due to the method according to the invention, there is no need to use stabilizers which are commonly used to provide sterical or electrostatic stabilization of suspensions or which increase the viscosity of suspensions. A suspension prepared by the method of the invention stays stable for a long period, at various pH values and even without being buffered.

In the context of the present invention, the term "suspension" refers to a mixture of a solid in a liquid or supercritical fluid. In other words, it means solid particles in a liquid medium. The terms "suspension" and "dispersion" are used interchangeably in the context of the present invention. The medium may be aqueous, partly aqueous or even nonaqueous. The medium may also be a supercritical fluid. Preferably, the medium is a liquid the pH of which can be set.

The term "stabilizers" as used herein means e.g. surface active agents, such as surfactants, thickeners *e.g.* hydrocolloids, poly-electrolytes, block co-polymers, and multivalent cations and anions, which are commonly used to provide sterical or electrostatic stabilization of suspensions.

In the context of the present invention, the term "intrinsic surface charge" refers to the "natural" surface charge of a particle, *i.e.* the surface charge which has not been changed by the addition of stabilizers. The surface charge of a particle can be measured by methods known in the art such as e.g. titration.

The term '"stable" in the context of stable suspension refers to a suspension in which particle aggregation is substantially or completely prevented, *i.e.* to a suspension wherein the mean particle size over a period of at least 1, 2, 3, 4, 5, 6, 7, 8, 9, 10, 15, 20, 25, or 30 days after size reduction, *e.g.* by grinding, does not increase more than a factor 10, 9, 8, 7, 6, 5, 4, 3, 2, more preferably the mean particle size increase during this period should be less than a factor 1.5, 1.3, 1.2, 1.1, 1.05, 1.01, 1.005, 1.001. In a preferred embodiment the particle size does not increase all. In the present invention, stability is achieved by wet milling at a pH at which the intrinsic surface charge of the compound is sufficient to minimize aggregation, *i.e.* at a pH at which aggregation is impeded. The skilled person can think of several methods for determining the suitable pH. In one embodiment, the suitable pH is determined by (i) wet milling at distinct pH values, (ii) determining at which pH the mean particle size does not increase more than a factor 10, 9, 8, 7, 6, 5, 4, 3, or 2 over a period of at least 1, 2, 3, 4, 5, 6, 7, 8, 9, 10, 15, 20, 25, or 30 days after grinding, and then (iii) selecting that pH for wet milling conditions. The selected pH is the suitable pH, *i.e.* the pH at which micronised particles can be produced without the need of using stabilizers to stabilize the suspension. The suitable pH can be a range of pH values (for instance pH$\leq$4 or pH$\geq$8 or pH between 5 and 9, to give just some examples), meaning that not one specific pH value is suitable, but several pH values within a range are suitable to prepare stable suspension of micronised particles. In an embodiment the invention provides for a method for preparing a stable suspension of micronised particles of a solid organic compound in the absence of a stabilizer according to the invention, the method comprising the steps of *e.g.* a) subjecting the solid organic compound to wet grinding at distinct pH values, b) determining the mean particle size of the obtained size-reduced solid organic compound over a period of at least 1, 2, 3, 4, 5, 6, 7, 8, 9, 10, 15, 20, 25, or 30 days after wet grinding, and c) preparing the stable suspension of micronised particles of the solid organic compound by subjecting the solid organic compound to size reduction by wet grinding under pH conditions, wherein the mean particle size of the obtained size-reduced solid organic compound does not increase more than a factor 10, 9, 8, 7, 6, 5, 4, 3, 2, more preferably the mean particle size increase should be less than a factor 1.5, 1.3, 1.2, 1.1, 1.05, 1.01, 1.005, 1.001 over a period of at least 1, 2, 3, 4, 5, 6, 7, 8, 9, 10, 15, 20, 25, or 30 days after wet grinding.

The skilled person may use the zeta-potential, which is related to the intrinsic surface charge, to get an indication of the suitable pH range. The significance of zeta potential is that its value can be related to the stability of suspensions. Compounds with a "high" zeta potential are electrically stabilized. To identical suspensions applies, the larger the absolute value of the zeta-potential, the higher the absolute value of the effective surface charge and the more stable the suspension. The zeta-potential may be measured using methods known in the art, for example by using a zeta-potential analyzer as described in Hunter R.J. (1998), Introduction to modern colloid science, Oxford Science press, page 241-247.

Preferably, the method according to the invention, *i.e.* the grinding, is performed at low ionic strength, *i.e.* at an ionic strength lower than 0.50 M, 0.40 M, 0.30 M or 0.20 M, more preferably, lower than 0.10 M or 0.05 M, even more preferably lower than 0.01 M (see e.g. Hunter R.J. (1998), Introduction to modern colloid science, Oxford Science press, page 207).

In the context of the present invention, the term "micronised particles" refers to particles with a mean diameter of 10 $\mu$m or less. In a preferred embodiment the diameter of the particles is 5 $\mu$m or less, 3 $\mu$m or less, 1 $\mu$m or less, 0.9 $\mu$m or less, 0.8 $\mu$m or less, 0.7 $\mu$m or less, 0.6 $\mu$m or less, 0.5 $\mu$m or less, 0.4 $\mu$m or less, 0.3 $\mu$m or less, and more preferably 0.2 $\mu$m or less. Most preferably, it refers to particles with a diameter of 0.1 $\mu$m or less, 0.075 $\mu$m or less, 0.050 $\mu$m or less or even 0.040 $\mu$m or less. Particle size is determined on the basis of the mean particle size, which in the context of the present invention is expressed as $d_{4,3}$ as measured by laser diffraction techniques *e.g.* light scattering. Light scattering returns a signal that is translated into an equivalent sphere diameter. Light scattering equipment is well-known in the art, for example a Beckmann Coulter Laser Diffraction Particle Size Analyzer called LS230 which uses laser light scattering coupled with wide angle measurements of scattered light. Preferably, at least 50%, 60%, 70%, 80%, 90%, 95%, 97% and most preferably 99% (w/w) or more of the particles in a suspension have the mean diameter equal to or smaller than the mean diameter given above. Other particle sizing techniques, such as microscopy, Scanning Electron Microscopy (SEM), Transition Electron Microscopy (TEM), Halo LM10 (Nanosight), Coulter Counter Multisizer 3 (Beckman Coulter) can of course also be used to determine the particle size.

The size reduction is achieved by wet grinding. Grinding techniques are well-known in the art and include media milling, such as bead milling, vibratory milling, ball milling; jet milling, for example using a microniser, a jet-o-mill or a jet pulveriser; dispersion milling and colloid milling; disc attrition milling; and homogenization. The mills and homogenizers used may have cooling equipment for maintaining the temperature below a value at which the materials to be milled decompose or otherwise lose activity and may have equipment to keep the materials to be milled under a controlled atmosphere.

In the context of the present invention, the phrase "wet grinding" refers to the process of exerting mechanical forces onto a solid material of interest that is dispersed or suspended in a liquid or fluid (in case of supercritical fluids) phase in order to break the solid material into smaller fragments (*i.e.* particle size reduction). Mechanical forces for wet milling can be applied in many ways and there are many different techniques available for wet milling. "Wet grinding" as used herein also includes homogenization techniques such as techniques, wherein a force which is caused by a large pressure drop over a small area is applied onto the solid material. In the present context, the terms "milling" and "grinding" are used interchangeably. In an embodiment of the invention the wet grinding in the method according to the invention is preceded by dry milling.

The wet grinding in the method according to the invention may be performed with or without the use of a grinding medium. In one embodiment of the invention, the wet grinding is performed using a grinding medium. In the context of the present invention, the term "grinding medium" refers to spherical beads from plastic, such as polystyrene; metal, such as zirconium oxide or steel; or glass, which are known in the art to be used for grinding compounds. Grinding media may have different hardness and density depending on the material used. Metal grinding media may be stabilized, for example with yttrium. Mixtures of beads from different composition may be used. In one embodiment, a grinding medium of zirconium oxide stabilized with yttrium is used.

The size of the beads in the grinding medium may vary. In the method according to the invention, beads with a mean diameter in the range from a few micrometers up to several millimeters are used. Mixtures of beads from different sizes may also be used in the method of the invention. In one embodiment, 0.3, 0.5 and 0.8 mm zirconium oxide beads stabilized with yttrium (Tosoh Corporation, Japan) are used.

In the context of the present invention, the term "organic compound" refers to any member of the large class of compounds whose molecules contain carbon and hydrogen; therefore, carbides, carbonates, carbon oxides and elementary carbon are not organic as meant herein. Semi-synthetic derivatives of organic compounds are also included in the present invention. The method of the invention may conveniently be used for poorly soluble organic compounds. In the context of this invention, a compound is poorly soluble in a specific solvent, if it has a solubility of less than 30 g/l at room temperature in that specific solvent, such as in for example organic solvents, such as ethanol, in oil or in water. Preferably, the organic compound is a food product, food supplement, feed product, agricultural product or a pharmaceutical compound, such as an organic pigment e.g. a carotenoid *e.g.* beta carotene, apocarotenal or its ester, lycopene, lutein, zeaxanthin, canthaxanthin or astaxanthin; an antibiotic *e.g.* a beta lactam; a vitamin; a benzoquinone *e.g.* coenzyme Q10; a protein; an enzyme; a peptide; a lipid; a polysaccharide; or a polyene antibiotic *e.g.* natamycin, nystatin, Amphotericin B, trienin, etruscomycin, filipin, chainin, dermostatin, lymphosarcin, candicidin, aureofungin A, aureofungin B, hamycin A, hamycin B or lucensomycin. In yet another embodiment the organic compound is coffee, cacao, grain/corn, starch granules to name just a few.

In the context of the present invention, the term "natamycin" refers to any type of natamycin, which compound is also known as pimaricin, and which is a polyene antifungal compound. Natamycin is well-known in the art, see for example EP 0678241; US 5,942,611; US 5,591,438 and EP 0600983.

The suspension of micronised particles obtained using the method according to the invention may subsequently be treated to physically separate the particles and the grinding medium, in order to remove the grinding medium from the end product. This may be done by any method known in the art for separating solids from a suspension including centrifugation, filtration and sedimentation, to name just a few.

The suspension of micronised particles obtained by the method of the invention may eventually be formulated into a product by the addition of additives and compounds known in the art for formulating a product, such as the addition of thickeners, surfactants and polymers. In a preferred embodiment the suspension of micronised particles obtained by the method of the invention is stable. The formulation of the suspension into a product may also involve the drying of the suspension. The suspension may be dried by any method known in the art which does not damage the product. In one embodiment, the suspension is dried by spray drying, vacuum drying, spray cooling, modified spray drying, sheet drying, crushing, freeze drying or drying processes that use supercritical $CO_2$ or any other supercritical fluidum.

In a further aspect the invention pertains to a suspension of micronised particles of a solid organic compound obtainable according to a method according to the invention. Preferably, the suspension is stable and substantially free of stabilizers, preferably stabilizer-free. The suspension may be liquid but may also have been subjected to drying and thus be dried *e.g.* a powder.

In another aspect of the invention, the suspension of micronised particles produced by the method according to the invention is used in a method for producing a powder, a coating, a film, or a foil. This method comprises using a suspension of micronised particles and formulating the suspension into, a concentrated suspension, a paste, a powder, a film, a coating or a foil using state of the art methods known to the skilled person. A concentrated suspension, paste, powder, film, coating or foil obtainable by the method is another aspect of the present invention. Using the method of the invention, a dispersable powder may be obtained which yields micronised particles upon dispersion in a liquid. Such a dispersible powder is also encompassed by the present invention. The obtained dispersion with micronised particles is stable under substantially stabilizer-free conditions.

The micronised particles, be it in the form of a suspension, a concentrated suspension, a paste, a powder, a coating, a film or a foil or any other suitable form, may be used in the food or feed industry, in horticulture, in agriculture or in the pharmaceutical industry. In one embodiment, they are used in or on a dairy product, such as on cheese and in cheese coatings. In another embodiment, they are used in or on fruit, mushroom growth substrate or spawn, potatoes, bulbs and on plants and flowers. In yet another embodiment, the suspension or other forms are used in pharmaceutical formulations, such as in the production of capsules and tablets, where they can be used as active ingredient or as excipient or additive, for example, in formulations for controlled release. In one embodiment, a suspension is used in the coating, as a color additive or a pigment.

A food product, feed product, horticultural product, agricultural product or pharmaceutical product comprising a suspension of micronised particles according to the invention or a concentrated suspension, a paste, a powder, a film, a coating or a foil according to the invention are another part of the present invention.

## EXAMPLES

### Example 1

### Measurement of particle size distribution and mean particle size of different organic compounds

Measurements were carried out for the following organic compounds: natamycin (DSM Gist, Delft, the Netherlands), nystatin (DSM, Delft, the Netherlands), cocoa (Blooker Cocoa, Amsterdam,the Netherlands) and coffee (Douwe Egberts Coffee, Utrecht, the Netherlands). For natamycin two products each containing differently shaped natamycin particles were included in the example; a natamycin product comprising disc-shaped particles and a natamycin product comprising needle-shaped particles. Before use the coffee was filtered through a 150 $\mu$m sieve. Samples of the above organic compounds were dispersed in demineralised water until the light obscuration value, which was measured by Laser Diffraction technology using a Laser Diffraction Particle Size Analyzer LS230 from BeckmanCoulter, Fullerton, CA, USA, was between 45% and 55%. This obscuration value was reached at a particle concentration of approx. 1% w/w. Then, the particle size distribution was analysed with the device for 90 seconds. The device made use of a red laser and a tungsten lamp with wavelengths of 450, 600 and 900 nm to measure the particle size. The device required the refractive index of the liquid phase and the particles and the imaginary refractive index of the particles for determining the particle size distribution from the raw data. These values were manually entered into the measurement device (refractive index liquid: 1.33; refractive index natamycin: 1.6; nystatin: 1.6; cocoa: 2.0; coffee: 2.0; imaginary refractive index natamycin: 0.01; nystatin: 0.01; cocoa: 1.0; coffee: 1.0). From the particle size distribution the mean particle size was determined. The mean particle size provided by the measurement device was a volume based particle size, $d_{4,3}$. The distribution per particle size range is given as a percentage of the total.

It follows from Table 1 that most particles of the disc-shaped natamycin product have a diameter between 10.0 and 50.0 $\mu$m, while most particles of the needle-shaped natamycin product have a diameter in the range of 5.0 to 10.0

μm. Most particles of nystatin have a diameter in the range of 2.0 to 5.0 μm. Most cocoa particles have a diameter in the range of 10.0 to 50.0 μm. Most coffee particles have a diameter in the range of 50.0 to 200.0 μm. Based on the particle size distribution the mean diameter of the different organic compounds was calculated. The mean particle size ($d_{4,3}$) of the particles of the disc-shaped natamycin product is 14.6 μm; the mean particle size ($d_{4,3}$) of the particles of the needle-shaped natamycin product is 6.4 μm; the mean particle size ($d_{4,3}$) of the particles of the nystatin product is 3.4 μm; the mean particle size ($d_{4,3}$) of the particles of the cocoa product is 22.6 μm; the mean particle size ($d_{4,3}$) of the particles of the coffee product is 76.5 μm.

Table 1. Particle size distribution of particles from various organic compounds.

| Particle size (μm) | Natamycin disc (% w/w) | Natamycin needle (% w/w) | Nystatin (% w/w) | Cocoa (% w/w) | Coffee (% w/w) |
|---|---|---|---|---|---|
| 0-0.2 | 0.0 | 10.2 | 7.4 | 0.0 | 0.0 |
| 0.2 - 0.8 | 0.0 | 7.2 | 4.4 | 0.6 | 0.3 |
| 0.8 - 2.0 | 0.8 | 10.5 | 27.2 | 2.8 | 1.1 |
| 2.0 - 5.0 | 18.4 | 24.9 | 46.7 | 9.2 | 1.2 |
| 5.0 - 10.0 | 32.6 | 31.7 | 12.2 | 19.1 | 1.6 |
| 10.0 - 50.0 | 47.8 | 15.6 | 2.1 | 64.0 | 35.9 |
| 50.0 - 200.0 | 0.4 | 0.0 | 0.0 | 4.3 | 59.8 |

## Example 2

**Preparation of a suspension of micronised natamycin particles in a ball mill using grinding medium at various grinding times**

A buffer solution of pH 8 was made by mixing 13.75 ml citric acid solution of 0.1 mol/l with 486.25 ml sodium hydroxide solution of 0.2 mol/l. 25 g of disc-shaped natamycin ($d_{4,3}$:14.6 μm; DSM Gist, Delft, the Netherlands) was added to the buffer solution and the powder was dispersed by means of stirring. The grinding chamber (300 ml) of the ball mill (Dynomill KDL Special from Bachofen AG, Basel, Switzerland) was filled up to 75% (v/v) with 0.3 mm zirconium oxide beads stabilized with yttrium (Tosoh Corporation, Tokyo, Japan). The ball mill was operated at 2000 rotations per minute. The natamycin suspension was pumped through the ball mill at a flow rate of 6.1 g/s. The suspension that exited the ball mill was returned to the vessel containing the rest of the suspension. The suspension passed through the chamber of the ball mill multiple times during the grinding experiment. The residence time of the suspension in the grinding chamber and the vessel was 26.7 seconds and 59.4 seconds, respectively. Samples of the suspension exiting the mill were taken after 2, 5, 10, 20, 30, 45, 60, 75 and 90 minutes. The particle size in the suspension samples was measured for each sample by means of laser diffraction using the Laser Diffraction Particle Size Analyzer called LS230 from BeckmanCoulter (Fullerton, CA, USA). The mean particle size that was measured is shown in Table 2.

Table 2: Mean particle size ($d_{4,3}$) of micronised natamycin particles as a function of the grinding time.

| Time (min) | $d_{4,3}$ (μm) |
|---|---|
| 0 | 14.58 |
| 2 | 8.57 |
| 5 | 5.69 |
| 10 | 2.90 |
| 20 | 1.12 |
| 30 | 0.45 |
| 45 | 0.37 |
| 60 | 0.21 |
| 75 | 0.18 |
| 90 | 0.18 |

## Example 3

**The influence of the bead size of the grinding medium and the rotation speed on mean particle size**

The experiment described in Example 2 was repeated with the same type of grinding medium. In addition, grinding media with bead sizes of 0.5 mm and 0.8 mm and a rotation speed of 3000 rpm, 4500 rpm and 6000 rpm were used. The results in Table 3 show that the grinding time is decreased when the bead size is smaller and/or the rotation speed is higher. This implies that particle size reduction is faster, in case the grinding media bead size is smaller and/or the rotation speed is higher.

Table 3. Required grinding time (in minutes) to obtain natamycin particles with a mean particle size of 0.18 μm.

| Rotation speed (rpm) / Grinding medium size (mm) | 0.3 | 0.5 | 0.8 |
|---|---|---|---|
| 2000 | 60-75 | 140-180 | 240-270 |
| 3000 | 40-50 | 120-135 | 180-210 |
| 4500 | 30-40 | 105-120 | 150-180 |
| 6000 | 20-25 | 45-60 | 120-140 |

## Example 4

**Influence of buffer system used during grinding on mean particle size**

The experiment described in Example 2 was repeated, with the proviso that natamycin was suspended in water. The pH of the obtained micronised natamycin suspension was 5.5. The mean particle size after grinding was 0.18 μm.
The experiment described in Example 2 was also repeated when using a buffer of dinatrium hydrogenphosphate (0.20 mol/l) and citric acid (0.10 mol/l) at different pH values ranging from 6 to 8. The results in Table 4 show that the pH in the studied range does not significantly influence the final mean particle size (0.18 μm) and that the time to reach the final particle was essentially equal for all pH values. This indicates that the pH has no significant effect on the grinding efficiency.

Table 4. Influence of pH on the mean particle size and the grinding time.

| pH | 6 | 7 | 8 |
|---|---|---|---|
| Grinding time (minutes) | 75-90 | 60-75 | 60-75 |
| Mean particle size $d_{4,3}$ (μm) | 0.18 | 0.18 | 0.18 |

## Example 5

**Particle size reduction using a homogenizer**

Disc-shaped natamycin powder was dispersed in water at a concentration of 3000 ppm. The mean particle size of the natamycin suspension was approximately 15 μm. The suspension was homogenized with a homogenizer (Panda 2K, ns100L, GEA NIRO SOAVI, Cell disrupter (R- type)) and a suspension comprising micronised natamycin particles was obtained. In Table 5 the mean particle size ($d_{4,3}$) of the particles of the obtained suspension is shown as a function of homogenization pressure for a different number of homogenization cycles. The results in Table 5 show that the mean particle size can be reduced significantly with a homogenizer and that particle size reduction is enhanced by increasing the homogenization pressure and the number of cycles through the homogenizer.

Table 5: Mean particle size as function of homogenizer pressure and number of cycles.

| Homogenization pressure (bar) | Number of cycles | Mean particle size ($\mu$m) |
|---|---|---|
| 750 | 3 | 3.8 |
| 1200 | 5 | 2.2 |
| 1500 | 25 | 2.0 |

### Example 6

**Determination of the zeta-potential of micronised natamycin suspensions**

A suspension comprising micronised natamycin was prepared essentially as described in Example 2, but grinding was carried out in demineralized water without pH correction with acid or base. The micronised product sample was added to an aqueous solution containing 1 mM KCl and aqueous solutions containing 0.1% (w/w) of the micronised product were obtained. The pH of the suspension was adjusted by means of titration with 0.25 M HCl and 0.10 M NaOH in the zeta-potential measurement device (automated system). The pH was varied in the range from pH 3 to 10 with steps of 1 $\pm$ 0.2 pH unit. The zeta-potential was measured for every pH value in a Zetasizer (Malvern, Worcestershire, United Kingdom). Results are shown in Table 6. From the results can be deducted that the isoelectric point of the micronised natamycin is at around pH 5.4. The results show that a suitable pH for forming stable micronised natamycin suspensions by wet grinding in the absence of a stabilizer can be found at a pH of around 4.6 or lower (zeta potential about +10 mV or higher or at a pH around 6.7 or higher (zeta potential about -20 mV or lower).

Table 6: Zeta potential of micronised natamycin suspensions at various pH values.

| pH | zeta-potential (mV) |
|---|---|
| 3 | +40 |
| 4 | +21 |
| 5 | +3 |
| 6 | -8 |
| 7 | -24 |
| 8 | -39 |
| 9 | -49 |
| 10 | -53 |

### Example 7

**Sedimentation properties of micronised and non-micronised natamycin suspensions**

In this experiment the stability of micronised and non-micronised natamycin suspensions was analyzed by observing the sedimentation behavior of the suspensions. A micronised natamycin suspension was prepared essentially as described in Example 2 with the proviso that the natamycin was suspended in demineralized water and the pH was not set. Next, a suspension with a final natamycin concentration of 1% (w/w) was prepared in an aqueous solution containing 0.01 M NaCl. The pH of samples of the suspension was set with 0.01 M NaOH and 0.01 M HCl. The samples of the suspension were stored and the pH was adjusted after 10 and 24 hours, if necessary. After 24 hours the pH of the samples of the suspension remained constant. Ten ml of each separate sample was placed in a plastic pipette (Ten ml sterile and disposable plastic pipette; inner diameter 7 mm; Bibby Sterilin Ltd. Stone, Staffordshire, United Kingdom) which was closed at both ends and stored vertically at 4°C. The sedimentation behavior of the samples was followed in time by measuring the volume above and below the sedimentation front within each pipette.

In Table 7 the results are shown. The percentages indicate the volume fraction below the sedimentation front. The table shows that the volume percentages below the sedimentation front decrease in time at certain pH conditions. This implies that the particles form sediment. The rate at which this occurs varies with pH. For example, at pH 3 and pH 10 the sedimentation is not significant, while at e.g. pH 4 the sedimentation is significant.

In addition to the sedimentation properties of a micronised natamycin suspension, the sedimentation properties of the starting material, *i.e.* non-micronised natamycin having a particle size of around 15 μm, at the pH values indicated in Table 7 was determined. Within 24 hours all particles of the suspension at all the different pH values (pH 3 to 10) did completely sediment. This is in contrast to the sedimentation behavior of any of the samples of the micronised natamycin suspension, wherein hardly any sedimentation was observed after 24 hours (see Table 7). It can therefore be concluded that the sedimentation rate of the micronised natamycin suspension is significantly decreased compared to that of the non-micronised natamycin suspension.

Comparison of the results in Table 7 with the zeta-potential measured in Example 6 shows that the sedimentation rate decreases when the difference between the pH and the iso-electric point (pl) becomes larger. This behaviour may be explained by the increase in electrostatic repulsion with the increase in the difference between pH and pl.

Table 7: Sedimentation behaviour of micronised natamycin.

| Time (days) | 0 | 0.5 | 1 | 2 | 3 | 4 | 5 | 6 | 7 | 8 |
|---|---|---|---|---|---|---|---|---|---|---|
| pH 3 | 100% | 100% | 100% | 100% | 100% | 100% | 100% | 99% | 98% | 98% |
| pH 4 | 100% | 96% | 97% | 88% | 87% | 85% | 83% | 81% | 79% | 77% |
| pH 5 | 100% | 97% | 92% | 85% | 79% | 75% | 72% | 70% | 68% | 66% |
| pH 6 | 100% | 98% | 94% | 89% | 83% | 79% | 76% | 73% | 70% | 68% |
| pH 7 | 100% | 98% | 94% | 89% | 84% | 80% | 77% | 74% | 72% | 71% |
| pH 8 | 100% | 100% | 98% | 96% | 93% | 92% | 90% | 88% | 86% | 84% |
| pH 9 | 100% | 100% | 99% | 97% | 96% | 95% | 93% | 92% | 90% | 89% |
| pH 10 | 100% | 100% | 100% | 100% | 100% | 100% | 100% | 100% | 100% | 99% |

## Example 8

### Preparation of a dispersible powder of a suspension comprising micronised natamycin particles

A micronised natamycin suspension was prepared in water essentially as described in Example 4. The particles of the suspension had a mean particle size of 0.18 μm. Twenty ml of the micronised natamycin suspension was frozen in liquid nitrogen before being placed under high vacuum for 24 hours. This freeze-drying process resulted in a dry powder. A representative sample of this powder was redispersed in water by means of stirring at a very low force and the obtained suspension was introduced into the LS230 Laser Diffraction Particle Size Equipment (BeckmanCoulter, Fullerton, CA, USA). The suspension was pumped continuously through the equipment and the particle size distribution was measured. The results of this measurement are shown in Table 8. The results demonstrate that granules of micronised particles made by wet grinding and subsequent drying (by *e.g.* freeze-drying) can, at least partly, re-disperse into the original (*i.e.* micronised) particle suspension when put in a liquid medium e.g. water.

Table 8: Mean particle size and amount of particles having diameter < 0.5 μm.

| Time (minutes) | Mean particle size $d_{4,3}$ (μm) | % (w/w) of particles having diameter < 0.4 μm |
|---|---|---|
| 0 | 13.8 | 6.6 |
| 2 | 13.4 | 10.9 |
| 4 | 12.6 | 15.5 |
| 7 | 10.2 | 20.6 |
| 11 | 8.5 | 34.0 |
| 15 | 4.2 | 49.6 |
| 17 | 0.46 | 84.2 |
| 19 | 0.45 | 84.7 |
| 36 | 0.40 | 87.7 |

(continued)

| Time (minutes) | Mean particle size $d_{4,3}$ ($\mu$m) | % (w/w) of particles having diameter < 0.4 $\mu$m |
|---|---|---|
| 40 | 0.40 | 87.5 |
| 50 | 0.39 | 88.2 |
| 61 | 0.34 | 90.7 |
| 70 | 0.31 | 92.6 |
| Micronised natamycin particles | 0.18 | 100.0 |

### Example 9

**Dissolution test of micronised natamycin**

A micronised natamycin suspension prepared essentially as described in Example 7 (with size smaller than 0.2 $\mu$m) was diluted 70 times with water at room temperature. The suspension was stirred. Samples were taken out after 5 seconds and then every 10 seconds. The particles were removed from the suspension by filtration with a 0.02 $\mu$m syringe-filter (Whatman International Ltd, England). The natamycin concentration of the remaining solution was measured with a spectrophotometer (Uvikon 933, Kontron Instruments). The results show that the micronised particles dissolve very fast. The solution reached saturation within 5 seconds (approximately 43 ppm natamycin in solution and solid particles remaining). The same test was repeated with non-grinded disc-shaped natamycin particles with a mean particle size of around 15 $\mu$m. In this case the saturation concentration was reached after approximately 2 hours.

In an alternative experiment the dissolution behaviour of different natamycin suspensions was analysed by suspending a known amount of natamycin particles in 500 ml of phosphate buffer solution (0.01 M, pH 6.8). The obtained suspension was stirred at 300 rpm with a magnetic stirrer. Samples of the suspension were taken in time. These samples were filtered with a 0.02 $\mu$m syringe filter (Whatman International Ltd, England) to remove the suspended natamycin particles. Then, the natamycin concentration was analysed in the filtrate by means of UV-light spectrophotometry (Uvikon 933, Kontron Instruments) at a wavelength of 304 nm. A calibration curve was made to translate the absorbance that was measured with the spectrophotometer into the natamycin concentration in ppm. HPLC analysis was used as a second natamycin quantification method. For this measurement methanol was added to the filtrate (1:1 in volume). The mixture was then loaded onto the HPLC column for quantification of natamycin.

In Table 9 the results are shown for different concentrations of three types of natamycin suspensions: suspensions comprising natamycin particles having a mean particle size of about 15 $\mu$m; suspensions comprising natamycin particles having a mean particle size of about 6 $\mu$m; and suspensions comprising micronised natamycin particles having a mean particle size of about 0.18 $\mu$m. These mean particle sizes were measured with the Laser Diffraction method as described before (LS230, Beckman Coulter; see Introduction to modern Colloid Science, Robert J. Hunter (1993), Oxford Science Publications).

The solutions that were analysed were:

A: Suspension of 15 $\mu$m disc-shaped natamycin particles (concentration: 837 ppm)
B: Suspension of 15 $\mu$m disc-shaped natamycin particles (concentration: 94.5 ppm)
C: Suspension of 6 $\mu$m needle-shaped natamycin particles (concentration: 765 ppm)
D: Suspension of 6 $\mu$m needle-shaped natamycin particles (concentration: 76.5ppm)
E: Suspension of micronised natamycin particles (concentration: 1185 ppm)
F: Suspension of micronised natamycin particles (concentration: 719 ppm)
G: Suspension of micronised natamycin particles (concentration: 118 ppm)
H: Suspension of micronised natamycin particles (concentration: 74 ppm)
I: Suspension of micronised natamycin particles (concentration: 50 ppm)
J: Suspension of micronised natamycin particles (concentration: 23 ppm)
K: Suspension of micronised natamycin particles (concentration: 5 ppm)

Table 9: Dissolved natamycin concentration over time (in min) for three types of natamycin particles at various natamycin suspension concentrations (concentrations are in ppm).

| Sample | 0 (min) | 1 (min) | 5 (min) | 10 (min) | 30 (min) | 60 (min) | 120 (min) | 180 (min) | 300 (min) |
|--------|---------|---------|---------|----------|----------|----------|-----------|-----------|-----------|
| A | 0 | 20 | 27 | 28 | 30 | 31 | 32 | 34 | 33 |
| B | 0 | ND | 5 | 15 | 21 | 22 | 24 | 24 | 28 |
| C | 1 | ND | 35 | 33 | 34 | 39 | 43 | 39 | 43 |
| D | 0 | 16 | 23 | 25 | 26 | 29 | 32 | 32 | 32 |
| E | 1 | 45 | 49 | 46 | 49 | 49 | 49 | 49 | 49 |
| F | 1 | 45 | 43 | 45 | 46 | 47 | 46 | 48 | 48 |
| G | 0 | 38 | 40 | 42 | 41 | 42 | 44 | 44 | 42 |
| H | 0 | 34 | 36 | 37 | 36 | 38 | 40 | 40 | 41 |
| I | 0 | 30 | 32 | 32 | 32 | 32 | 32 | ND | ND |
| J | 0 | 19 | 20 | 20 | 20 | 20 | 20 | 20 | 21 |
| K | 0 | 5 | 5 | 5 | 5 | 5 | 5 | 5 | ND |
| ND means not determined | | | | | | | | | |

Comparison of dissolution behaviour of suspensions A and F shows that the dissolved natamycin concentration is higher for micronised natamycin particles than for 15 $\mu$m natamycin particles, even though the natamycin concentration is lower within the suspension comprising micronised natamycin particles (719 ppm vs 837 ppm).

Comparison of dissolution behaviour of suspensions C and F shows that the dissolved natamycin concentration is higher for micronised natamycin particles than for 6 $\mu$m natamycin particles, even though the natamycin concentration is lower within the suspension comprising micronised natamycin particles (719 ppm vs 765 ppm).

The results demonstrate that micronised natamycin particles have a higher solubility and solubilisation rate compared to the 6 $\mu$m and 15 $\mu$m natamycin particles. This can be beneficial especially in applications wherein a high dissolved natamycin concentration is required.

**Example 10**

**Preparation of suspensions of micronised nystatin particles in a ball mill using a grinding medium at various grinding times.**

The experiment was essentially performed as for natamycin (see Example 2). The particle size that was obtained is shown in Table 10. Similar mean particle sizes were obtained when the experiment was repeated in a dinatrium hydrogen phosphate/citric acid buffer having pH 5 ($d_{4,3}$: 0.17 $\mu$m after grinding time of 240-270 minutes) or pH 8 ($d_{4,3}$: 0.17 $\mu$m after grinding time of 210-240 minutes) or when the experiment was repeated in demineralized water ($d_{4,3}$: 0.17 $\mu$m).

Table 10: Mean particle size ($d_{4,3}$) of micronised nystatin particles as a function of the grinding time.

| Time (min) | $d_{4,3}$ ($\mu$m) |
|------------|---------------------|
| 0 | 3.42 |
| 5 | 1.43 |
| 10 | 0.77 |
| 20 | 0.38 |
| 30 | 0.34 |
| 45 | 0.23 |
| 60 | 0.21 |

(continued)

| Time (min) | $d_{4,3}$ ($\mu$m) |
|---|---|
| 75 | 0.16 |
| 90 | 0.15 |
| 105 | 0.15 |

## Example 11

**Determination of the zeta-potential of micronised nystatin**

Nystatin was milled and the zeta-potential was measured essentially as described for natamycin in Example 6. Results are shown in Table 11. From the results can be deducted that the isoelectric point of the micronised nystatin is at around pH 4.6. From the results can be concluded that a suitable pH for forming stable micronised nystatin suspensions by wet grinding in the absence of a stabilizer can be prepared at a pH of about 4.4 or lower (zeta potential about 10 mV or higher) or a pH of about 5.8 or higher (zeta potential about -20 mV or lower).

Table 11: Zeta potential of suspensions of micronised nystatin at various pH values.

| pH | zeta-potential (mV) |
|---|---|
| 3 | +39 |
| 4 | +14 |
| 5 | -7 |
| 6 | -23 |
| 7 | -33 |
| 8 | -41 |
| 9 | -48 |
| 10 | -55 |

## Example 12

**Sedimentation properties of micronised nystatin suspensions**

The experiment was essentially performed as for natamycin (see Example 7). In Table 12 the results are shown. The percentages indicate the volume fraction below the sedimentation front. The table shows that the volume percentages below the sedimentation front decrease in time at certain pH conditions. This implies that the particles form sediment. The rate at which this occurs varies with pH. At pH 8 and higher the sedimentation is not significant, while at *e.g.* pH 4 sedimentation is much faster.

In addition to the sedimentation properties of the micronised nystatin suspensions, the sedimentation properties of the starting material, *i.e.* non-micronised nystatin having a particle size of around 3.4 $\mu$m, was determined at various pH values. Non-micronised nystatin suspensions at pH 4 to 6 had a volume percentage below the sedimentation front of around 25% after one day. This is in contrast to the sedimentation behavior of the micronised nystatin suspensions wherein hardly any sedimentation was observed after one day. It can therefore been concluded that the sedimentation rate of micronised nystatin suspensions is significantly decreased compared to that of non-micronised nystatin suspensions.

Comparison of the above results with the zeta-potential measured in Example 11 shows that the sedimentation rate decreases when the difference between the pH and the iso-electric point (pl) becomes larger. This behaviour may be explained by the increase in electrostatic repulsion with the increase in the difference between pH and pl.

Table 12: Sedimentation behaviour of micronised nystatin.

| Time (days) | 0 | 0.5 | 1 | 2 | 3 | 4 | 7 | 8 | 9 |
|---|---|---|---|---|---|---|---|---|---|
| pH 4 | 100% | 99.5% | 95.9% | 91.3% | 85.0% | 84.0% | 75.1% | 72.5% | 70.1% |

(continued)

| Time (days) | 0 | 0.5 | 1 | 2 | 3 | 4 | 7 | 8 | 9 |
|---|---|---|---|---|---|---|---|---|---|
| pH 5 | 100% | 99.2% | 97.0% | 94.0% | 89.5% | 88.9% | 84.0% | 82.9% | 81.6% |
| pH 6 | 100% | 100% | 99.1% | 97.8% | 95.1% | 94.9% | 91.5% | 90.4% | 89.1% |
| pH 7 | 100% | 100% | 99.9% | 99.4% | 98.1% | 97.9% | 95.0% | 93.0% | 91.0% |
| pH 8 | 100% | 100% | 99.9% | 99.7% | 99.0% | 98.8% | 96.8% | 96.1% | 95.7% |
| pH 9 | 100% | 100% | 100% | 100% | 100% | 100% | 100% | 100% | 100% |
| pH 10 | 100% | 100% | 100% | 100% | 100% | 100% | 100% | 100% | 100% |

## Example 13

### Challenge test using micronised natamycin

To test the efficacy of micronised natamycin in a cheese application, an experiment was performed wherein the surface of fresh brined Gouda cheeses were challenged with *Penicillium discolor* strains and treated with formulations comprising natamycin with different particle sizes. The amount of mould spores that was able to germinate on the treated cheese surfaces are an indication of the anti-fungal activity of the natamycin analyzed. The cheeses (10 per natamycin particle type) were considered to be no longer protected by natamycin, when at least 50% of the cheeses analyzed had more than two growing colonies.

Cheeses were coated with a coating containing base polymer Craymul 4386 (Cray Valley, Brummen) and containing natamycin particles. Cheeses were then contaminated at day zero with *Penicillium discolor* PED1, CR1A and *Penicillium discolor* PED74, L1 at a concentration of $1.1 \times 10^3/cm^2$. The cheeses were visually checked for fungal growth on a daily basis for three weeks. The experiment was carried out with three different natamycin particles: disc-shaped natamycin particles having a mean particle diameter of 15 $\mu$m; grinded disc-shaped natamycin particles for which grinding was stopped when the mean particle diameter was 1.23 $\mu$m; and micronised natamycin particles having a mean particle diameter of 0.18 $\mu$m. Grinding for both products was done in water; the pH of the suspension was 5.9; the pH was not set with acid or base. The concentration of natamycin in the coating was 150 ppm or 250 ppm for all natamycin particles. As a control, 10 cheeses comprising no natamycin in the coating were analyzed. These cheeses had an average protection time of 5 to 6 days. In Table 13 the results are shown.

From the results can be concluded that protection against fungal growth increases, when higher natamycin concentrations are used. Micronised natamycin particles provide the longest protection against mould growth. It is clear from the results of the experiment that a decrease in natamycin particle size increases protection against fungal growth.

Table 13. Protection of cheese against fungal growth in days.

| Amount natamycin (ppm) | Natamycin (15 $\mu$m) | Natamycin (1.23 $\mu$m) | Micronised natamycin (0.18 $\mu$m) |
|---|---|---|---|
| 150 | 16 | - | 21 |
| 250 | 18 | 21 | 25 |

## Example 14

### Preparation of a suspension of micronised cocoa particles in a ball mill using grinding medium

The preparation of the micronised particles was done essentially as described in Example 2 with the exception that Cocoa powder (Blooker Cocoa, Amsterdam, the Netherlands) was suspended in demineralized water to obtain a 1% (w/v) cocoa suspension. Hundred milliliter of the obtained suspension was introduced into the grinding chamber of the ball mill (Dynomill KDL Special from Bachofen AG, Basel, Switzerland) which was filled up to 75% (v/v) with 0.3 mm zirconium oxide beads stabilized with yttrium (Tosoh Corporation, Tokyo, Japan). The ball mill was operated at 2000 rotations per minute in batch mode. This implies that the bead mill was filled with the suspension without circulating the suspension through the vessel that was connected to the bead mill. The pH of the suspension after wet grinding was 6.4. After 16 hours of grinding a mean particle size ($d_{4,3}$) of 0.14 $\mu$m was obtained. The non-micronised cocoa product had a mean size of 22.6 $\mu$m, which indicates that the wet grinding resulted in a significant size reduction of the particles.

**Example 15**

**Sedimentation properties of micronised cocoa suspensions**

The experiment was essentially performed as for natamycin (see Example 7). At pH 5 and higher no significant sedimentation was observed within 72 hours. At pH 3 and 4 significant sedimentation was observed within 24 hours.

In addition to the sedimentation properties of the micronised cocoa suspensions, the sedimentation properties of the starting material, *i.e.* non-micronised cocoa having a particle size of around 23 $\mu$m was determined at similar pH values as were applied in Example 7. Within 24 hours all particles within the suspensions at all pH values did totally sediment. This is in contrast to the sedimentation behavior of the micronised cocoa suspensions wherein no significant sedimentation was observed after 72 hours at pH values of 5 and higher. It can therefore be concluded that the sedimentation rate of micronised cocoa suspensions is significantly decreased at certain pH values compared to that of non-micronised cocoa suspensions.

**Example 16**

**Preparation of a suspension of micronised coffee particles in a ball mill using grinding medium**

The preparation of the micronised particles was done essentially as described in Example 2 with the exception that coffee powder (Douwe Egberts Coffee, Utrecht, The Netherlands) was filtered through a 150 $\mu$m sieve and the fines were suspended in demineralized water to obtain a 1% (w/v) coffee suspension. Hundred milliliter of the obtained suspension was introduced into the grinding chamber of the ball mill (Dynomill KDL Special from Bachofen AG, Basel, Switzerland) which was filled up to 75% (v/v) with 0.3 mm zirconium oxide beads stabilized with yttrium (Tosoh Corporation, Tokyo, Japan). The ball mill was operated at 2000 rotations per minute in batch mode. This implies that the bead mill was filled with the suspension without circulating the suspension through the vessel that was connected to the bead mill. The pH of the suspension after wet grinding was 6.9. After 8 hours of grinding a mean particle size ($d_{4,3}$) of 31 $\mu$m was reached. The non-grinded coffee product had a mean size of 76.5 $\mu$m, which indicates that wet grinding resulted in a significant size reduction of the particles. Nevertheless, the mean particle size was still larger than for other products tested. However, a distinct fraction representing 5.7% w/w of the product was below 0.2 $\mu$m. For the non-grinded coffee particles none of the particles were smaller than 0.2 $\mu$m. This shows that by grinding a significant fraction of very small coffee particles can be produced. For natamycin and nystatin a clear effect of the grinding time on the reduction of the average particle size was observed. It may therefore be concluded that longer grinding times for coffee particles may result in a further reduction of the particle size.

Table 14. Particle size distribution of grinded and non-grinded coffee particles

| Particle size ($\mu$m) | Non-grinded coffee (% w/w) | Grinded coffee (% w/w) |
|---|---|---|
| 0 - 0.2 | 0.0 | 5.7 |
| 0.2 - 0.8 | 0.3 | 3.0 |
| 0.8 - 2.0 | 1.1 | 2.7 |
| 2.0 - 5.0 | 1.2 | 6.6 |
| 5.0 - 10.0 | 1.6 | 12.9 |
| 10.0 - 50.0 | 35.9 | 59.1 |
| 50.0 - 200.0 | 59.8 | 10.2 |

**Example 17**

**Sedimentation properties of grinded coffee suspensions**

The experiment was essentially performed as for natamycin (see Example 7). The grinded product formed sediment under certain pH conditions. At pH 5 and higher no significant sedimentation was observed within 24 hours. At lower pH, e.g. pH 3 and pH 4 sedimentation was significant within 24 hours.

In addition to the sedimentation properties of the micronised coffee suspensions, the sedimentation properties of the starting material, *i.e.* non-micronised coffee having a particle size of around 76 $\mu$m was determined at similar pH values as were applied in Example 7. Within 24 hours all particles within the suspensions at all pH values did totally sediment. This is in contrast to the sedimentation behavior of the micronised coffee suspensions wherein no significant sedimentation was observed after 24 hours at pH values of 5 and higher. It can therefore been concluded that the sedimentation rate of micronised coffee suspensions is significantly decreased at certain pH values compared to that of non-micronised coffee suspensions.

**Claims**

1. A method for preparing a stable suspension of micronised particles of natamycin in the absence of a stabilizer, the method comprising the steps of:

   a) subjecting the natamycin to wet grinding at distinct pH values to obtain suspensions of micronised particles of natamycin each having a distinct pH value, or subjecting natamycin to wet grinding at one pH value and adjusting the pH of the suspension after grinding to obtain suspensions of micronised particles of natamycin each having a distinct pH value,
   b) measuring the zeta potential of the obtained suspensions and determining which of the obtained suspensions has a zeta potential of 10 mV or higher or -20 mV or lower, and
   c) preparing a suspension of micronised particles of natamycin by subjecting the natamycin to size reduction by wet grinding under pH conditions, wherein the suspension has a zeta potential of 10 mV or higher or -20 mV or lower according to step b).

2. A method according to claim 1, **characterized in that** the suspension comprises an ionic strength lower than 0.50 M.

3. A method according to claim 1 or 2, **characterized in that** the wet grinding in step a) and/or step c) is performed using a grinding medium.

4. A method according to anyone of the claims 1 to 3, **characterized in that** the wet grinding in step a) and/or step c is preceded by dry milling.

5. A method according to claim 3 or 4 which further comprises the step of physical separating the grinding medium and the micronised particles.

6. A method according to anyone of the claims 1 to 5 which further comprises the step of formulating the suspension of micronised particles of natamycin into a product.

7. A method according to anyone of the claims 1 to 6, **characterized in that** the micronised particles have a mean particle size of 1 $\mu$m or less.

8. A suspension of micronised particles of natamycin, obtainable by the method according to anyone of the claims 1 to 7.

9. Use of the suspension according to claim 8 to produce a concentrated suspension, a paste, a powder, a coating, a film or a foil.

10. A method for producing a concentrated suspension, a paste, a powder, a film, a coating or a foil comprising micronised particles of natamycin, which method comprises using a suspension of micronised particles according to claim 8.

11. A concentrated suspension, a paste, a powder, a film, a coating or a foil obtainable by the method according to claim 10.

12. A powder according to claim 11, which powder yields a stable suspension of micronised particles upon dispersion in a liquid.

13. Use of a suspension of micronised particles according to claim 8 or a concentrated suspension, a paste, a powder, a film, a coating or a foil according to claim 11 or 12 in the food or feed industry, in horticulture, in agriculture or in the pharmaceutical industry.

14. A food product, feed product, horticultural product, agricultural product or pharmaceutical product comprising a suspension of micronised particles according to claim 8 or a concentrated suspension, a paste, a powder, a film, a coating or a foil according to claim 11 or 12.

**Patentansprüche**

1. Verfahren zur Herstellung einer stabilen Suspension mikrovermahlener Natamycinpartikel, ohne dass ein Stabilisator vorliegt, wobei das Verfahren die folgenden Schritte umfasst:

   a) Nassvermahlen des Natamycins bei bestimmten pH-Werten, um Suspensionen mikrovermahlener Natamycinpartikel zu erhalten, die jeweils einen bestimmten pH-Wert aufweisen, oder Nassvermahlen von Natamycin bei einem einzigen pH-Wert und Einstellen des pH der Suspension nach dem Vermahlen, um Suspensionen mikrovermahlener Natamycinpartikel zu erhalten, die jeweils einen bestimmten pH-Wert aufweisen,
   b) Messen den Zeta-Potentials der erhaltenen Suspensionen und Bestimmen, welche der erhaltenen Suspensionen ein Zeta-Potential von mindestens 10 mV oder höchstens -20 mV aufweist, und
   c) Herstellen einer Suspension mikrovermahlener Natamycinpartikel, indem das Natamycin durch Nassvermahlen unter pH-Bedingungen eine Zerkleinerung erfährt, wobei die Suspension ein Zeta-Potential von mindestens 10 mV oder höchstens -20 mV gemäß Schritt b) aufweist.

2. Verfahren nach Anspruch 1, **dadurch gekennzeichnet, dass** die Suspension eine Ionenstärke von weniger als 0,50 M umfasst.

3. Verfahren nach Anspruch 1 oder 2, **dadurch gekennzeichnet, dass** das Nassvermahlen in Schritt a) und/oder Schritt c) unter Verwendung eines Mahlmediums durchgeführt wird.

4. Verfahren nach einem beliebigen der Ansprüche 1 bis 3, **dadurch gekennzeichnet, dass** dem Nassvermahlen in Schritt a) und/oder Schritt c) ein Trockenmahlvorgang vorangeht.

5. Verfahren nach Anspruch 3 oder 4, welches weiterhin den Schritt des physikalischen Trennens des Mahlmediums und der mikrovermahlenen Partikel umfasst.

6. Verfahren nach einem beliebigen der Ansprüche 1 bis 5, welches weiterhin den Schritt des Formulierens der Suspension mikrovermahlener Natamycinpartikel in ein Produkt umfasst.

7. Verfahren nach einem beliebigen der Ansprüche 1 bis 6, **dadurch gekennzeichnet, dass** die mikrovermahlenen Partikel eine mittlere Partikelgröße von höchstens 1 $\mu$m haben.

8. Suspension mikrovermahlener Natamycinpartikel, wobei diese durch das Verfahren nach einem beliebigen der Ansprüche 1 bis 7 erhalten werden kann.

9. Verwendung der Suspension nach Anspruch 8, um eine konzentrierte Suspension, eine Paste, ein Pulver, eine Beschichtung, eine Dünnschicht oder eine Folie herzustellen.

10. Verfahren zur Herstellung einer konzentrierten Suspension, einer Paste, eines Pulvers, einer Dünnschicht, einer Beschichtung oder einer Folie, die/das mikrovermahlene Natamycinpartikel umfasst, wobei das Verfahren die Verwendung einer Suspension mikrovermahlener Partikel gemäß Anspruch 8 umfasst.

11. Konzentrierte Suspension, Paste, Pulver, Dünnschicht, Beschichtung oder Folie, die/das durch das Verfahren nach Anspruch 10 erhalten werden kann.

12. Pulver nach Anspruch 11, wobei das Pulver eine stabile Suspension mikrovermahlener Partikel ergibt, wenn es in einer Flüssigkeit dispergiert wird.

13. Verwendung einer Suspension mikrovermahlener Partikel nach Anspruch 8 oder einer konzentrierten Suspension, einer Paste, eines Pulvers, einer Dünnschicht, einer Beschichtung oder einer Folie nach Anspruch 11 oder 12 in der Nahrungsmittel- oder Futtermittelindustrie, im Gartenbau, in der Landwirtschaft oder in der pharmazeutischen Industrie.

14. Nahrungsmittelprodukt, Futtermittelprodukt, Gartenbauprodukt, landwirtschaftliches Produkt oder pharmazeutisches Produkt, das eine Suspension mikrovermahlener Partikel nach Anspruch 8 oder eine konzentrierte Suspension, eine Paste, ein Pulver, eine Dünnschicht, eine Beschichtung oder eine Folie nach Anspruch 11 oder 12 umfasst.

**Revendications**

1. Procédé de préparation d'une suspension stable de particules micronisées de natamycine en l'absence d'un stabilisant, le procédé comprenant les étapes de :

   a) soumission de la natamycine à un broyage humide à des valeurs de pH distinctes pour obtenir des suspensions de particules micronisées de natamycine ayant chacune une valeur de pH distincte, ou soumission de la natamycine à un broyage humide à une seule valeur de pH et ajustement du pH de la suspension après broyage pour obtenir des suspensions de particules micronisées de natamycine ayant chacune une valeur de pH distincte,
   b) mesure du potentiel zêta des suspensions obtenues et détermination de laquelle des suspensions obtenues a un potentiel zêta de 10 mV ou plus ou -20 mV ou moins, et
   c) préparation d'une suspension de particules micronisées de natamycine par soumission de la natamycine à une réduction de taille par broyage humide dans des conditions de pH, la suspension ayant un potentiel zêta de 10 mV ou plus ou -20 mV ou moins selon l'étape b).

2. Procédé selon la revendication 1, **caractérisé en ce que** la suspension comprend une force ionique inférieure à 0,50 M.

3. Procédé selon la revendication 1 ou 2, **caractérisé en ce que** le broyage humide dans l'étape a) et/ou l'étape c) est effectué au moyen d'un milieu de broyage.

4. Procédé selon l'une quelconque des revendications 1 à 3, **caractérisé en ce que** le broyage humide dans l'étape a) et/ou l'étape c) est précédé par un broyage à sec.

5. Procédé selon la revendication 3 ou 4 qui comprend en outre l'étape de séparation physique du milieu de broyage et des particules micronisées.

6. Procédé selon l'une quelconque des revendications 1 à 5 qui comprend en outre l'étape de formulation de la suspension de particules micronisées de natamycine dans un produit.

7. Procédé selon l'une quelconque des revendications 1 à 6, **caractérisé en ce que** les particules micronisées ont une taille de particule moyenne de 1 $\mu$m ou moins.

8. Suspension de particules micronisées de natamycine, pouvant être obtenue par le procédé selon l'une quelconque des revendications 1 à 7.

9. Utilisation de la suspension selon la revendication 8 pour produire une suspension concentrée, une pâte, une poudre, un revêtement, un film ou une feuille.

10. Procédé de production d'une suspension concentrée, une pâte, une poudre, un film, un revêtement ou une feuille comprenant des particules micronisées de natamycine, ledit procédé comprenant l'utilisation d'une suspension de particules micronisées selon la revendication 8.

11. Suspension concentrée, pâte, poudre, film, revêtement ou feuille pouvant être obtenu par le procédé selon la revendication 10.

12. Poudre selon la revendication 11, ladite poudre produisant une suspension stable de particules micronisées après dispersion dans un liquide.

13. Utilisation d'une suspension de particules micronisées selon la revendication 8 ou une suspension concentrée, une pâte, une poudre, un film, un revêtement ou une feuille selon la revendication 11 ou 12 dans l'industrie alimentaire humaine et animale, en horticulture, en agriculture ou dans l'industrie pharmaceutique.

14. Produit d'alimentation humaine, produit d'alimentation animale, produit d'horticulture, produit d'agriculture ou produit pharmaceutique comprenant une suspension de particules micronisées selon la revendication 8 ou une suspension concentrée, une pâte, une poudre, un film, un revêtement ou une feuille selon la revendication 11 ou 12.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 9835666 A
- WO 9106292 A
- US 2876160 A
- US 4006025 A
- US 5858410 A
- WO 2007014566 A
- EP 0807431 A
- GB 1148801 A
- EP 0678241 A
- US 5942611 A
- US 5591438 A
- EP 0600983 A

**Non-patent literature cited in the description**

- **YOKOYAMA T. et al.** *KONA,* 2005, vol. 23, 7-17
- **CAMP T.R.** Sedimentation and the design of settling tanks. *Trans. Am. Soc. Civ. Eng.,* 1946, vol. 111, 895-958
- **DOBBINS W.E.** Effect of turbulence on sedimentation. *Trans. Am. Soc. Civ. Eng.,* 1944, vol. 109, 629-653
- **HUNTER R.J.** Introduction to modern colloid science. Oxford Science press, 1998, 241-247
- **HUNTER R.J.** Introduction to modern colloid science. Oxford Science press, 1998, 207
- **ROBERT J. HUNTER.** Introduction to modern Colloid Science. Oxford Science Publications, 1993